# EUROPEAN PATENT SPECIFICATION

(11) **EP 3 006 929 B1**
(45) Date of publication and mention of the grant of the patent: **22.03.2023**
(21) Application number: 15191728.3
(22) Date of filing: 29.06.2005
(51) Int. Cl.: G01N 27/22

(54) **ELECTRIC FIELD SENSOR**
ELEKTRIKFELDSENSOR
CAPTEUR DE CHAMP ELECTRIQUE

(30) Priority: 29.06.2004 CA 2472865
(43) Date of publication of application: 13.04.2016
(62) Divisional of application: 05761895.1
(73) Proprietor: Instrumar Limited, St. John, Newfoundland A1B 4A5 (CA)
(72) Inventor: Inkpen, Stuart, Portugal Cove-St. Phillips, NL, A1M 1C5 (CA); Nolan, Chris, St. John's, Newfoundland and Labrador A1A 4B5 (CA); Pike, Darryl, St. John's, Newfoundland and Labrador A1E 2E2 (CA); Rowe, Heather, St. John's, Newfoundland and Labrador A1A 5S9 (CA); Hall, John, St. John's, Newfoundland and Labrador A1E 2B1 (CA); Linfield, Dana, Portugal Cove-St. Philips, NL, A1M 2N4 (CA); Dawson, Chris, St. John's, Newfoundland and Labrador A1E 5V7 (CA); Galway, Gerard, Torbay, Newfoundland and Labrador A1K 1L2 (CA); Walsh, Shawn, St. John's, Newfoundland and Labrador A1B 2C5 (CA); Abraham, Ruth, St. John's, Newfoundland and Labrador A1A 4B6 (CA); Swamidas, Joshua, St. John's, Newfoundland and Labrador A1B 4S1 (CA)
(74) Representative: Hartig, Michael

(56) References cited:
- EP-A1- 0 665 952
- DE-C1- 10 203 816
- JP-A- S61 195 341
- US-A1- 2003 052 867

## Description

### FIELD OF THE INVENTION

The present invention relates to an electric field sensor.

### BACKGROUND OF THE INVENTION

In textile production, synthetic fibres are formed from a "spin-draw" process in which a molten polymer, such as polyester or nylon, is spun into filaments, and twisted together to form a single fibre. The spun fibre is then drawn, altering the fibre's elasticity, tensile strength and diameter. During the process, typically a liquid emulsion or "finish" is applied to the fibre to lubricate the filaments and thereby reduce static electricity generated by the movement of the fibre through processing machinery. Further, interlacing nodes are typically formed in the fibre by exposing the drawn fibre to a pressurized airjet, thereby bonding the individual filaments together at periodic intervals along the fibre.

Lack of uniformity in fibre bulk, finish, denier, or interlacing node distribution can cause fibre entanglement or breakage, or irregularities in fibre coloration during the weaving process, resulting in costly production-line shutdowns for the end-user. Accordingly, attempts have been made to monitor the physical characteristics of fibre in real-time, as it is being produced, to identify defects in the fibre before it is shipped to the end-user.

For instance, Fabbri (US 4,706,014 ) and Meyer (US 5,394,096 ) use a capacitive sensor to respectively measure the diameter and denier of a polymer fibre. However, capacitive sensors can only detect large variations in denier. Further, it is not possible to monitor other fibre characteristics of importance to textile users, such as finish, bulk, node count and node quality, using a capacitive sensor.

Sakai (US 4,491,831 ) uses a phototransistor to detect yarn irregularities. The phototransistor generates an analog signal in response to yarn unevenness. The analog signal is digitized, and then subjected to real-time frequency analysis, to thereby detect both cyclic and non-cyclic yarn irregularities. However, it is not possible to monitor other fibre characteristics of importance to textile users, such as finish, bulk, node count and node quality, using a phototransistor.

Felix (US 4,888,944 ) monitors a pair of process parameters, such as yarn tension and speed, to detect changes in denier, filament breakage, and absence of finish. However, using the disclosed monitored parameters, it would not be possible to monitor other fibre characteristics of importance to textile users, such as bulk, node count and node quality.

Instrumar Ltd. ( CA 2,254,426 ) uses an electric field sensor for measuring physical fibre characteristics in real-time. Changes in the physical characteristics of a fibre as it is drawn past the sensor causes a current to be induced in the electrode. Comparing the changes in magnitude and phase of the induced current against known fibre profiles allows Instrumar to monitor the denier, finish and interlacing of the drawn fibre in real-time. However, the electrode is sensitive to changes in electric field adjacent to the fibre, thereby reducing the sensitivity of the sensor to the desired fibre characteristics. Further, it is not possible to monitor other fibre characteristics of importance to textile users, such as bulk and node quality, using the described sensor measurements.

### SUMMARY OF THE INVENTION

An electric field sensor includes a plurality of electrodes and a plurality of conductors coupled to the electrodes. The conductors are capable of receiving a sinusoidal voltage signal and are capable of outputting a current induced in the electric field sensor.

The electric field sensor comprises an insulating substrate having a pair of opposing surfaces. The electrodes are disposed on one of the opposing surfaces of the substrate and extend parallel to each other on the one opposing surface. Each electrode has an electrode end.

The electrodes comprise a plurality of first electrodes, and a plurality of second electrodes interleaved and non-contacting with the plurality of first electrodes on the one opposing surface. The plurality of first electrodes are not in contact with each other on the one opposing surface and extend across the one opposing surface from one end of the substrate. The plurality of second electrodes are not in contact with each other on the one opposing surface and extend across the one opposing surface from an opposite end of the substrate.

The conductors extend from the one opposing surface through to the other of the opposing surfaces of the substrate.

The conductors comprise a plurality of first conductors and a plurality of second conductors. Each of the first conductors are coupled to the electrode end of a respective one of the first electrodes proximate the one substrate end. Each of the second conductors are coupled to the electrode end of a respective one of the second electrodes proximate the opposite substrate end.

The conductors may comprise vias that extend at a right angle to the electrodes.

The electric field sensor may also include an insulator disposed over the electrodes. The insulator may comprise one of ceramic and glass. The ceramic may comprise alumina.

### BRIEF DESCRIPTION OF THE DRAWINGS

The present invention will now be described, by way of example only, with reference to the accompanying drawings, in which:
- Fig. 1 is a schematic view of the computer-based fibre production monitoring system, according to the present invention, depicting the sensors, the sensor monitor, the computer server and a measurements database;
- Fig. 2a is a schematic view of one of the sensors (an electric field sensor) depicted in Fig. 1 ;
- Fig. 2b is a top plan view of the electric field sensor depicted in Fig. 2a ;
- Figs. 2c and 2d are transverse cross-sectional views of the electric field sensor;
- Fig. 3a is a schematic view of the structure of the data packet created by the sensor processing unit; and
- Fig. 3b is a schematic view of the structure of the data record created by the sensor monitor.

### DETAILED DESCRIPTION OF THE PREFERRED EMBODIMENT

### 1. Fibre Production Monitoring System: Overview

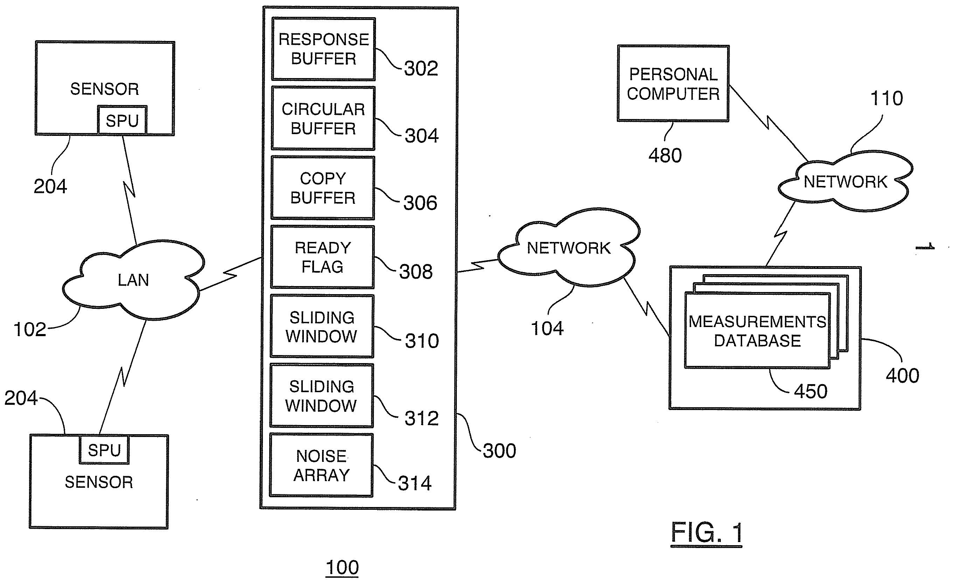

Turning now to Fig. 1 , there is shown a computer-based fibre production monitoring system, denoted generally as 100, comprising a plurality of sensors 200, a sensor monitor 300, a computer server 400, a local area network 102 interconnecting the sensors 200 and the sensor monitor 300, and a communications network 104 interconnecting the sensor monitor 300 and the computer server 400.

Preferably, the local area network 102 comprises a DeviceNet bus, although other network protocols may be used. Preferably the communications network 104 comprises a wired local area Ethernet network. However, the communications network 104 can also utilize other network protocols, and can comprise a wide area network, or a wireless network. Further, although the fibre production monitoring system 100 is shown including a number of sensors 200, the fibre production monitoring system 100 need only include a single sensor 200.

### 2. Sensor

Each sensor 200 is typically disposed on a threadline of multi-bobbin fibre production line.

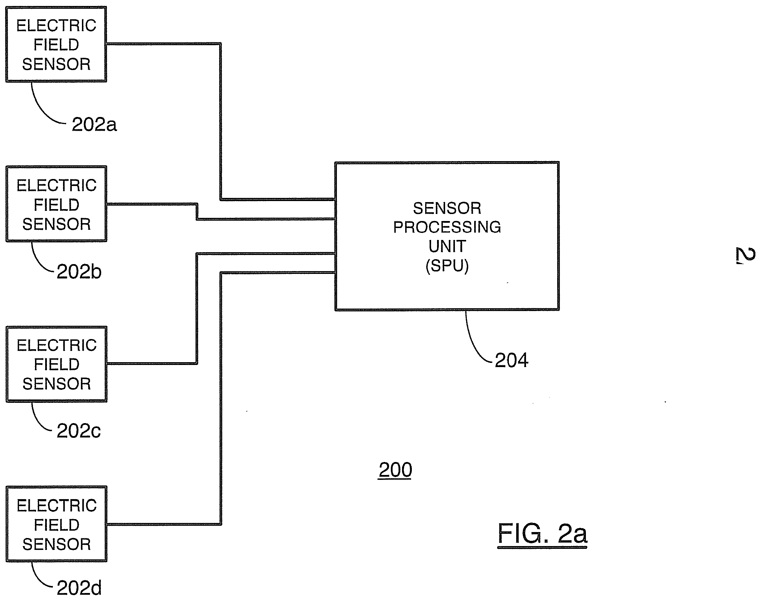

Each sensor 200 may monitor the physical characteristics of a number of the multi-filament fibres, as the fibres are drawn past the sensor 200, prior to being wound on the bobbin. As shown in Fig. 2a , preferably the sensor 200 comprises a plurality of electric field sensors 202 (depicted as 202a, 202b, 202c, 202d), and a sensor processing unit (SPU) 204 coupled to the electric field sensors 202. Each electric field sensor 202 may monitor the physical characteristics of one of the multi-filament fibres. However, as will be appreciated, the sensor 200 need not include several electric field sensors 202, but instead could include only a single electric field sensor 202.

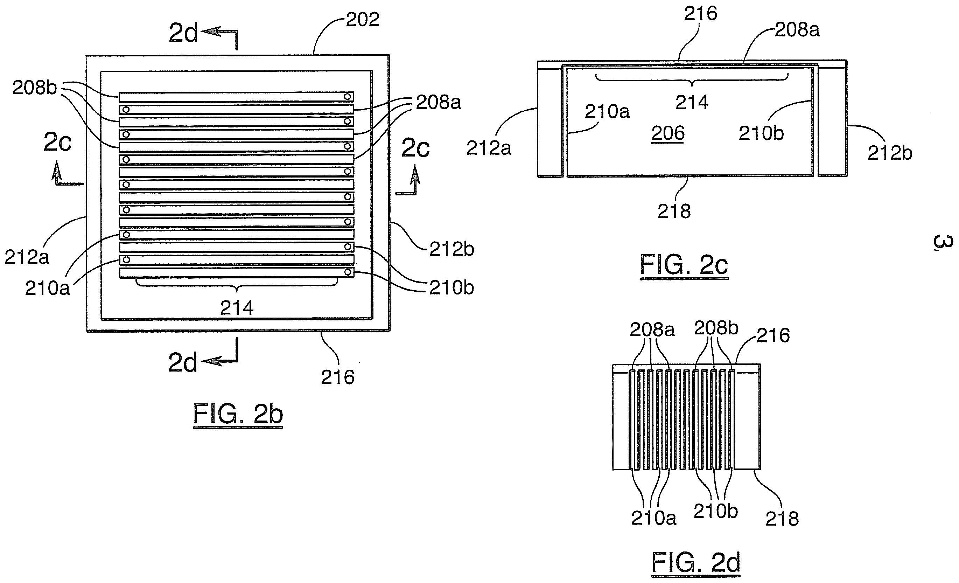

As shown in Figs. 2b, 2c and 2d, each electric field sensor 202 comprises an insulating substrate 206, a plurality of electrodes 208 disposed on the substrate 206, and a plurality of vias 210 extending downwardly through the substrate 206. Preferably, the substrate 206 comprises a non-conductive material, such as ceramic, although other non-conductive materials may be used. Also, preferably the electrodes 208 are substantially planar and are formed on the substrate 206 using conventional printed circuit board or integrated circuit manufacturing techniques. The electrodes 208 extend across the top surface of the substrate 206 in a substantially parallel fashion, such that the electrodes 208 do not contact one another on the top surface of the substrate 206.

The electrodes 208 are segregated into a first electrode portion 208a, and a second electrode portion 208b. The electrodes 208 of the first electrode portion 208a extend from one end 212a of the substrate 206, and the electrodes 208 of the second electrode portion 208b extend from the opposite end 212b of the substrate 206. The electrodes 208 of the first electrode portion 208a are interlaced with the electrodes 208 of the second electrode portion 208b in the centre region 214 of the top surface of the substrate 206.

Typically, each via 210 comprises a plated through-hole extending from one end of a respective electrode 208, through the substrate 206, to the bottom 218 of the sensor 202. Alternately, the vias 210 maybe provided as conductive traces or wires extending in a similar manner. The vias 210 are segregated into a first via portion 210a, and a second via portion 210b. The vias 210 of the first via portion 210a are coupled to the first electrode portion 208a, and the vias 210 of the second via portion 210b are coupled to the second electrode portion 208b. Each via 210 is connected to a respective electrode 208 adjacent the respective end 212, and extends from the electrode 208 through the substrate 206, from the top surface thereof to the bottom surface 218 thereof. With this configuration, the sensitivity of the electric field sensor 202 to electric fields outside the centre region 214 is less than prior art electric field sensors.

Preferably, the vias extend at a right angle to the electrodes, through the substrate 206. The electric field sensor 202 preferably also includes an insulator layer 216 disposed over the electrodes 208. Typically, the insulator layer 216 comprises ceramic or glass, although the ceramic alumina is preferred due to its hardness. Preferably, the electric field sensor 202 includes guides (not shown) extending upwards from the insulator layer 216, within the centre region 214, which guide the drawn fibre in a direction transverse to the orientation to the electrodes 208.

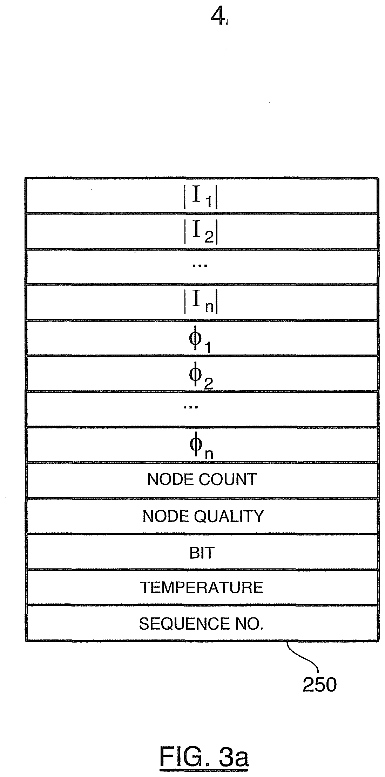

The sensor processing unit (SPU) 204 includes a number of data buses, each connected to the vias 210 of a respective one of the electric field sensors 202. Each via 210 connects to a respective conductor of the data buses at the bottom 216 of the sensor 202. The SPU 204 applies a respective sinusoidal voltage signal to the electrode portions 208, with the voltage signal applied to the first electrode portion 208a being complementary (ie 180 degrees out of phase) to the voltage signal applied to the second electrode portion 208b. The SPU 204 also monitors the current induced in each electric field sensor 202, as the associated fibres are drawn past the electric field sensors 202. The SPU 204 includes an internal A/D converter that periodically digitizes the current measurements from the associated electric field sensors 202. Based on the current measurements, the SPU 204 creates a data packet 250 (see Fig. 3a ) that includes a series of measurements of physical characteristics of the fibre as it is drawn past the electric field sensor 202.

As shown in Fig. 3a, each data packet 250 includes a series of magnitude measurements and a series of phase measurements, measured over a predetermined measurement time span.

### 3. Sensor Monitor

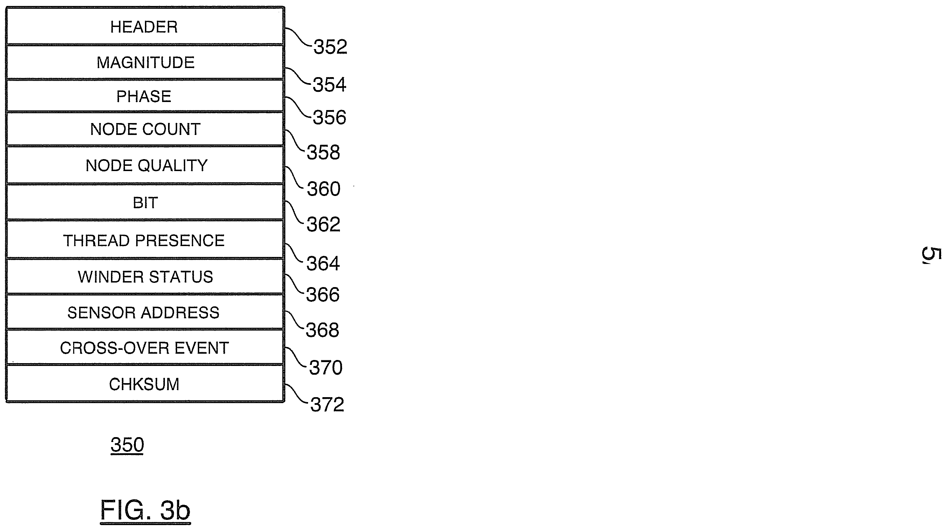

The sensor monitor 300 is coupled to the sensor processing units 204 of the sensors 200 via the DeviceNet bus 102. Preferably, the sensor monitor 300 includes a DeviceNet scanner that periodically sends out commands to the sensor processing units 204, requesting data packets 250 from the sensors 200. As will be explained, the sensors 200 provide the sensor monitor 300 with the data packets 250 for the associated threadlines, which the sensor monitor 300 converts into data records 350 (see Fig. 3b) and transmits to the computer server 400 over the communications network 104. The DeviceNet scanner includes a response buffer 302 (Fig. 1) that is used to store the data packets 250 prior to being converted into the data records 350.

### 4. Fibre Production Monitoring System: Method of Operation

The method of operation of the fibre production monitoring system 100 will now be described.

The sensor processing units 204 apply the aforementioned complementary sinusoidal voltage signals to the associated electric field sensor(s) 202, and continuously measure the current induced in the associated electric field sensor(s) 202 as the fibre is drawn past the electric field sensor(s) 202. Each sensor processing unit 204 measures the induced current over a predetermined measurement time span.

At the end of each time span, each sensor processing unit 204 assembles a data packet 250 from the measured currents. The data packet 250 includes the series of current magnitude measurements and the series of current phase measurements, measured over the measurement time span.

The DeviceNet scanner of the sensor monitor 300 transmits read commands to the sensor processing units 204, requesting the data packets 250 from the sensor processing units 204, receives a data packet 250 from the sensor processing unit 204, and stores the received data packet 250 into the memory of the response buffer 302.

When the sensor monitor 300 detects the presence of a new data packet 250 in the response buffer 302, the sensor monitor 300 removes the data packet 250 from the response buffer 302, and creates a data record 350 from the removed data packet 250.

Upon receipt of the data records 350, the computer server 400 compares the measurements contained therein against defined parameter limits. If one of the measurements deviates from the range established by the defined parameter limits, the computer server 400 activates an audible and/or visual alarm.

## Claims

1. An electric field sensor (202) including a plurality of electrodes (208) and a plurality of vias (210) coupled to the electrodes (208), the vias (210) being capable of receiving a sinusoidal voltage signal and being capable of outputting a current induced in the electric field sensor (202), wherein the electric field sensor (202) comprises:
an insulating substrate (206) having a pair of opposing surfaces;
the electrodes (208) are disposed on one of the opposing surfaces of the substrate and extend parallel to each other on the one opposing surface, each said electrode (208) having an electrode end, the electrodes comprising a plurality of first electrodes (208a) and a plurality of second electrodes (208b) interleaved and non- contacting with the plurality of first electrodes (208a) on the one opposing surface, wherein the plurality of first electrodes (208a) are not in contact with each other on the one opposing surface and extend across the one opposing surface from one end of the substrate, and the plurality of second electrodes (208b) are not in contact with each other on the one opposing surface and extend across the one opposing surface from an opposite end of the substrate; and
the vias (210) extend from the one opposing surface through to the other of the opposing surfaces (218) of the substrate, wherein the vias comprise a plurality of first vias (210a) and a plurality of second vias (210b), wherein each of the first vias (210a) are coupled to the electrode end of a respective one of the first electrodes (208a) proximate the one substrate end, and each of the second vias (210b) are coupled to the electrode end of a respective one of the second electrodes (208b) proximate the opposite substrate end.

2. The electric field sensor (202) according to claim 1, wherein the vias (210) comprise vias that extend at a right angle to the electrodes (208).

3. The electric field sensor (202) according to claim 1 or claim 2, further including an insulator (216) disposed over the electrodes (208).

4. The electric field sensor (202) according to claim 3, wherein the insulator (216) comprises one of ceramic and glass.

5. The electric field sensor (202) according to claim 4, wherein the ceramic comprises alumina.

## Patentansprüche

1. Elektrischer Feldsensor (202) mit einer Vielzahl von Elektroden (208) und einer Vielzahl von Vias (210), die mit den Elektroden (208) gekoppelt sind, wobei die Vias (210) in der Lage sind, ein sinusförmiges Spannungssignal zu empfangen, und in der Lage sind, einen in dem elektrischen Feldsensor (202) induzierten Strom auszugeben, wobei der elektrische Feldsensor (202) aufweist:
ein isolierendes Substrat (206) mit einem Paar gegenüberliegender Oberflächen; die Elektroden (208) auf einer der gegenüberliegenden Oberflächen des Substrats angeordnet sind und sich parallel zueinander auf der einen gegenüberliegenden Oberfläche erstrecken, wobei jede Elektrode (208) ein Elektrodenende aufweist, wobei die Elektroden eine Vielzahl von ersten Elektroden (208a) und eine Vielzahl von zweiten Elektroden (208b) aufweisen, die mit der Vielzahl von ersten Elektroden (208a) auf der einen gegenüberliegenden Oberfläche verschachtelt sind und diese nicht berühren, wobei die mehreren ersten Elektroden (208a) auf der einen gegenüberliegenden Fläche nicht miteinander in Kontakt sind und sich von einem Ende des Substrats über die eine gegenüberliegende Fläche erstrecken, und die mehreren zweiten Elektroden (208b) auf der einen gegenüberliegenden Fläche nicht miteinander in Kontakt sind und sich von einem gegenüberliegenden Ende des Substrats über die eine gegenüberliegende Fläche erstrecken; und
die Vias (210) sich von der einen gegenüberliegenden Oberfläche durch die andere der gegenüberliegenden Oberflächen (218) des Substrats erstrecken, wobei die Vias eine Vielzahl von ersten Vias (210a) und eine Vielzahl von zweiten Vias (210b) aufweisen, wobei jedes der ersten Vias (210a) mit dem Elektrodenende einer jeweiligen der ersten Elektroden (208a) in der Nähe des einen Substratendes gekoppelt ist, und jedes der zweiten Vias (210b) mit dem Elektrodenende einer jeweiligen der zweiten Elektroden (208b) in der Nähe des gegenüberliegenden Substratendes gekoppelt ist.

2. Elektrischer Feldsensor (202) nach Anspruch 1, wobei die Vias (210) Vias aufweisen, die sich in einem rechten Winkel zu den Elektroden (208) erstrecken.

3. Elektrischer Feldsensor (202) nach Anspruch 1 oder Anspruch 2, der ferner einen über den Elektroden (208) angeordneten Isolator (216) aufweist.

4. Elektrischer Feldsensor (202) nach Anspruch 3, wobei der Isolator (216) entweder Keramik oder Glas aufweist.

5. Elektrischer Feldsensor (202) nach Anspruch 4, wobei die Keramik Aluminiumoxid aufweist.

## Revendications

1. Capteur de champ électrique (202) comprenant une pluralité d'électrodes (208) et une pluralité de trous d'interconnexion (210) couplés aux électrodes (208), les trous d'interconnexion (210) étant en mesure de recevoir un signal de tension sinusoïdal et étant en mesure de fournir en sortie un courant induit dans le capteur de champ électrique (202), dans lequel le capteur de champ électrique (202) comprend :
un substrat isolant (206) présentant une paire de surfaces opposées ;
dans lequel les électrodes (208) sont disposées sur l'une des surfaces opposées du substrat et s'étendent parallèlement les unes aux autres sur ladite une surface opposée, chaque dite électrode (208) présentant une extrémité d'électrode, les électrodes comprenant une pluralité de premières électrodes (208a) et une pluralité de secondes électrodes (208b) intercalées et sans contact avec la pluralité de premières électrodes (208a) sur la surface opposée, dans lequel les électrodes de la pluralité de premières électrodes (208a) ne sont pas en contact les unes avec les autres sur ladite une surface opposée et s'étendent à travers ladite une surface opposée à partir d'une extrémité du substrat, et les électrodes de la pluralité de secondes électrodes (208b) ne sont pas en contact les unes avec les autres sur ladite une surface opposée et s'étendent à travers ladite une surface opposée à partir d'une extrémité opposée du substrat ; et
les trous d'interconnexion (210) s'étendent de ladite une surface opposée à l'autre des surfaces opposées (218) du substrat, dans lequel les trous d'interconnexion comprennent une pluralité de premiers trous d'interconnexion (210a) et une pluralité de seconds trous d'interconnexion (210b), dans lequel chacun des premiers trous d'interconnexion (210a) est couplé à l'extrémité d'électrode d'une électrode respective des premières électrodes (208a) à proximité de ladite une extrémité du substrat, et chacun des seconds trous d'interconnexion (210b) est couplé à l'extrémité d'électrode d'une électrode respective des secondes électrodes (208b) à proximité de l'extrémité opposée du substrat.

2. Capteur de champ électrique (202) selon la revendication 1, dans lequel les trous d'interconnexion (210) comprennent des trous d'interconnexion qui s'étendent à un angle droit par rapport aux électrodes (208).

3. Capteur de champ électrique (202) selon la revendication 1 ou 2, comprenant en outre un isolant (216) disposé sur les électrodes (208).

4. Capteur de champ électrique (202) selon la revendication 3, dans lequel l'isolant (216) comprend un élément parmi de la céramique et du verre.

5. Capteur de champ électrique (202) selon la revendication 4, dans lequel la céramique comprend de l'alumine.
